# EUROPÄISCHE PATENTANMELDUNG

(11) **EP 1 170 278 A2**
(43) Veröffentlichungstag der Anmeldung: **09.01.2002**
(21) Anmeldenummer: 01113994.6
(22) Anmeldetag: 08.06.2001
(51) Int. Cl.: C07C 45/42, C07C 41/48

(54) **Verfahren zur Herstellung von Alkyl- oder Aryloxyacetaldehyden**

(30) Priorität: 05.07.2000 AT 11532000
(71) Anmelder: DSM Fine Chemicals Austria Nfg GmbH & Co KG, 4021 Linz (AT)
(72) Erfinder: Giselbrecht, Karlheinz, 4061 Pasching (AT); Hermanseder, Rudolf, 4624 Pennewang (AT)
(74) Vertreter: Klostermann, Ingrid

(57) **Zusammenfassung**

Verfahren zur Herstellung von Alkyl- oder Aryloxyacetaldehyden der Formel 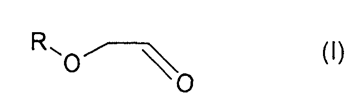 in der R einen gegebenenfalls ein- oder mehrfach substituierten Alkyl-, Aryl-, Heteroaryl-, Alkaryl-, Alkylheteroaryl- oder Aralkylrest oder einen gegebenenfalls ein- oder mehrfach substituierten Heterocyclus oder Alkylheterocyclus bedeuten kann, bei welchem eine Verbindung der Formel

R―OM (II)

in der R wie oben definiert ist und M ein Alkali- oder ein Erdalkaliatom sein kann mit einer Verbindung der Formel 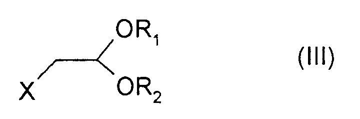 in der R₁ und R₂ unabhängig voneinander einen C₁-C₆-Alkylrest oder gemeinsam einen C₂-C₆-Alkylenrest und X ein Halogenatom bedeuten, zu dem entsprechenden Dialkylacetal der Formel 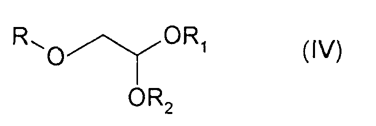 in der R, R₁ und R₂ wie oben definiert sind, umgesetzt wird, worauf eine Acetalspaltung zum gewünschten Alkyl- oder Aryloxyacetaldehyde der Formel (I) durchgeführt wird.

## Beschreibung

Die Erfindung betrifft ein Verfahren zur Herstellung von Alkyl- oder Aryloxyacetaldehyden aus den entsprechenden Alkoholaten über die Diacetale mit anschließender Acetalspaltung.

Alkyl- oder Aryloxyacetaldehyde sind wertvolle Ausgangsprodukte in der organischen Synthese. So finden sie beispielsweise als Edukt zur Herstellung von Pharma- und Agrarchemikalien Verwendung.

Zur Herstellung der Alkyl- oder Aryloxyacetaldehyden, wie etwa Benzyloxyacetaldehyd, sind bereits verschiedene Varianten in der Literatur beschrieben. Eine Möglichkeit stellt beispielsweise die in J. Org. Chem. (1997), 62(8), 2622-2624 beschriebene Metaperiodatspaltung von Diolen, wie etwa Glycerin, dar.
Eine Alternative ist die in Synth. Commun. (1988), 18(4), 359-66 beschriebene NalO₄-Spaltung von substituierten Glycerolacetoniden.
Die gewünschten Acyloxyacetaldehyde können gemäß J. Org. Chem. (1988), 53(18), 4274-82) aber auch ausgehend von beispielsweise 2-(Benzyloxy)ethanol durch eine Swern-Oxidation hergestellt werden.
Die Nachteile der bisher bekannten Herstellungsvarianten sind u.a. durch die Verwendung von kritischen Oxidationsmitteln, wie Periodat u.s.w., und/oder durch die schwer zugänglichen bzw. teuren Edukte bedingt.

Aufgabe der Erfindung war es, ein neues Verfahren zur Herstellung von Acyloxyacetaldehyden zu finden, das von leicht zugänglichen Edukten ausgeht und in wenigen, einfachen Schritten zu dem gewünschten Endprodukt führt.

Unerwarteterweise konnte diese Aufgabe durch Verwendung von Halogenacetaldehyddialkylacetalen und Alkoholaten als Ausgangsverbindungen gelöst werden.

Gegenstand der Erfindung ist demnach ein Verfahren zur Herstellung von Alkyl- oder Aryloxyacetaldehyden der Formel 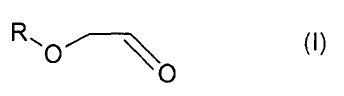 in der R einen gegebenenfalls ein- oder mehrfach substituierten Alkyl-, Aryl-, Heteroaryl-, Alkaryl-, Alkylheteroaryl- oder Aralkylrest oder einen gegebenenfalls ein- oder mehrfach substituierten Heterocyclus oder Alkylheterocyclus bedeuten kann, das dadurch gekennzeichnet ist, dass eine Verbindung der Formel

R―OM (II)

in der R wie oben definiert ist und M ein Alkali- oder ein Erdalkaliatom sein kann mit einer Verbindung der Formel 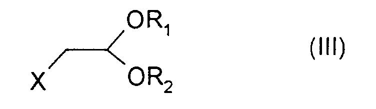 in der R₁ und R₂ unabhängig voneinander einen C₁-C₆-Alkylrest oder gemeinsam einen C₂-C₆-Alkylenrest und X ein Halogenatom bedeuten zu dem entsprechenden Dialkylacetal der Formel 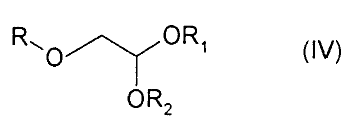 in der R, R₁ und R₂ wie oben definiert sind, umgesetzt wird, worauf eine Acetalspaltung zum gewünschten Alkyl- oder Aryloxyacetaldehyde der Formel (I) durchgeführt wird.

Bei dem erfindungsgemäßen Verfahren werden Alkyl- oder Aryloxyacetaldehyde der Formel (I) hergestellt.
In der Formel (I) bedeutet R einen gegebenenfalls ein- oder mehrfach substituierten Alkyl-, Aryl-, Heteroaryl-, Alkaryl-, Alkylheteroaryl- oder Aralkylrest oder einen gegebenenfalls ein- oder mehrfach substituierten Heterocyclus oder Alkylheterocyclus.

Unter Alkyl sind dabei gesättigte oder ein- oder mehrfach ungesättigte, lineare, verzweigte oder cyclische, primäre, sekundäre oder tertiäre Hydrocarbonreste zu verstehen. Dies sind beispielsweise C₁-C₂₀-Alkylreste, wie etwa Methyl, Ethyl, Propyl, iso-Propyl, Butyl, iso-Butyl, t-Butyl, Pentyl, Cyclopentyl, iso-Pentyl, neo-Pentyl, Hexyl, iso-Hexyl, Cyclohexyl, Cyclohexylmethyl, 3-Methylpentyl, 2,2-Dimethylbutyl, 2,3-Dimethylbutyl, Octyl, Cyclooctyl, Decyl, Cyclodecyl, Dodecyl, Cyclododecyl u.s.w. Bevorzugt sind dabei C₁-C₁₂-Alkylreste und besonders bevorzugt C₂-C₈-Alkylreste. Die Alkylgruppe kann gegebenenfalls ein oder mehrfach durch unter den Reaktionsbedingungen inerte Substituenten substituiert sein. Geeignete Substituenten sind beispielsweise Carbonsäureester oder -amide, Alkoxy, bevorzugt C₁-C₆-Alkoxy, Aryloxy, bevorzugt C₆-C₂₀-Aryloxy, Nitro, Cyano, Sulfonsäureester oder -amide u.s.w..

Unter Aryl sind bevorzugt C₆-C₂₀-Arylgruppen zu verstehen, wie etwa Phenyl, Biphenyl, Naphthyl, Indenyl, Fluorenyl u.s.w.
Die Arylgruppe kann dabei gegebenenfalls durch unter den Reaktionsbedingungen inerte Substituenten ein- oder mehrfach substituiert sein. Geeignete Substituenten sind dabei wiederum Carbonsäureester oder -amide, Alkoxy, bevorzugt C₁-C₆-Alkoxy, Aryloxy, bevorzugt C₆-C₂₀-Aryloxy, Nitro, Cyano, Sulfonsäureester oder - amide u.s.w..

Unter Alkaryl oder Alkylaryl sind Alkylgruppen zu verstehen, die einen Arylsubstituenten aufweisen, wie etwa Benzyl.
Aralkyl oder Arylalkyl bezieht sich auf eine Arylgruppe mit einem Alkylsubstituenten.

Unter Heteroaryl oder Heterocyclus sind cyclische Reste zu verstehen die zumindestens ein O- oder N-Atom im Ring enthalten. Dies sind beispielsweise Furyl, Pyridyl, Pyrimidyl, Imidazolyl, Tetrazolyl, Pyrazinyl, Benzofuranyl, Chinolyl, Isochinolyl, Isobenzofuryl, Pyrazolyl, Indolyl, Isoindolyl, Benzoimidazolyl, Purinyl, Carbazolyl, Oxazolyl, Isoxazolyl, Pyrrolyl, Chinazolinyl, Pyridazinyl, Phthalazinyl u.s.w.

Funktionelle O- oder N-Gruppen können dabei nötigenfalls geschützt werden.
Die Heteroarylgruppe bzw. der Heterocyclus kann dabei gegebenenfalls ein- oder mehrfach durch die bereits oben angeführten Substituenten substituiert sein.

Unter Alkylheteroaryl bzw. Alkylheterocyclus sind dabei Alkylgruppen zu verstehen, die durch eine Heteroarylgruppe bzw. durch einen Heterocyclus substituiert sind.

Bevorzugte Verbindungen der Formel (I) sind solche, in denen R einen gegebenenfalls ein- oder mehrfach substituierten C₁-C₁₂-Alkylrest, besonders bevorzugt einen C₂-C₈-Alkylrest oder einen Alkylarylrest mit 1-12 C-Atomen im Alkylteil, besonders bevorzugt Benzyl bedeuten.
Bevorzugte Substituenten sind Carbonsäureester oder -amide, C₁-C₆-Alkoxy, C₆-C₂₀-Aryloxy, Nitro oder Cyano.
Besonders bevorzugt ist der Rest R unsubstituiert.

Zur Herstellung der Verbindungen der Formel (I) werden erfindungsgemäß eine Verbindung der Formel (II) mit einer Verbindung der Formel (III) umgesetzt.
In der Formel (II) ist R wie in der Formel (I) definiert und M bedeutet ein Alkali- oder ein Erdalkaliatom. Bevorzugte Alkali- oder ein Erdalkaliatome sind dabei Li, Na, K, Ca, Mg, Cs. Besonders bevorzugt sind Na oder K.
Die Verbindungen der Formel (II) sind zumeist kommerziell in großen Mengen und preiswert erhältlich oder sie können auf einfachem Weg beispielsweise durch Umsetzung des entsprechenden Alkohols ROH mit einem Alkoholat MOAlkyl, beispielsweise mit Natriummethanolat, in einem Alkohol AlkylOH, beispielsweise Methanol, als Lösungsmittel hergestellt werden.

In der Formel (III) bedeuten R₁ und R₂ unabhängig voneinander einen C₁-C₆-Alkylrest, bevorzugt einen C₁-C₄-Alkylrest.
Der Alkylrest kann dabei gesättigt, linear, verzweigt oder cyclisch sein. Bevorzugt sind lineare oder verzweigte Alkylreste, wie Methyl, Ethyl, Propyl, i-Propyl, Butyl Hexyl. Besonders bevorzugt sind Methyl, Ethyl und Propyl.
R₁ und R₂ können aber auch gemeinsam einen C₂-C₆-Alkylenrest bedeuten, sodass ein cyclisches Acetal gebildet wird. C₂-C₆-Alkylenreste sind dabei Ethylen, Propylen, Butylen, Pentylen und Hexylen. Bevorzugt sind C₂-C₄-Alkylenreste.
X bedeutet in der Formel (III) Halogen.
Bevorzugt bedeutet X Cl oder Br; besonders bevorzugt Cl.

Die Verbindungen der Formel (II) und der Formel (III) werden erfindungsgemäß in äquimolarer Menge eingesetzt oder eine der beiden Verbindungen im molaren Überschuss, wobei bevorzugt die Verbindung der Formel (II) im molaren Überschuss von 1,1 bis 2 mol pro mol Verbindung der Formel (III) verwendet wird.

Die Umsetzung erfolgt gegebenenfalls in einem organischen Lösungsmittel. Geeignete Lösungsmittel sind solche die unter den Reaktionsbedingungen inert sind. Bevorzugt werden höhersiedende Lösungsmittel, wie etwa Xylol usw. eingesetzt.
Liegt die Verbindung der Formel (II) in flüssiger Form vor, so ist kein zusätzliches Lösungsmittel notwendig; die Verbindung der Formel (II) fungiert dabei sowohl als Edukt als auch als Lösungs- bzw. Verdünnungsmittel.
Bevorzugt wird die Reaktion ohne zusätzliches Lösungs- bzw. Verdünnungsmittel durchgeführt

Die Reaktionstemperatur hängt vom gegebenenfalls verwendeten Lösungsmittel, sowie von den Edukten ab und liegt zwischen 70 und 200°C, bevorzugt zwischen 80 und 180°C und besonders bevorzugt zwischen 100 und 160°C.

Anschließend wird dem Reaktionsgemisch bei 30 bis 100°C soviel Wasser zugegeben, bis sich die gesamte gegebenenfalls ausgefallene Menge an Salz MX löst. Durch diese Maßnahme werden gleichzeitig eventuell vorhandene Nebenprodukte in Form von Orthoestern verseift.
Weiters führt die Wasserzugabe zu einer Phasentrennung, nach der die leichtsiedenden Komponenten in Form von Alkohol R₁OH bzw. R₂OH, wie etwa Methanol oder Ethanol, Wasser, ROH und überschüssiger Verbindung der Formel (III) oder (II) abdestilliert werden. Im Sumpf verbleibt die Verbindung der Formel (IV), die gewünschtenfalls destillativ aufgereinigt werden kann.
Die Verbindung der Formel (IV) ist in den meisten Fällen, wie etwa im Fall von Benzyloxyacetaldehyd, sehr stabil und wird in hoher Reinheit erhalten. Durch die Stabilität kann die Verbindung der Formel (IV) über längere Zeit gelagert werden, sodass die Acetalspaltung zur Verbindung der Formel (I) nicht sofort durchgeführt werden muss, sondern bei Bedarf erfolgen kann.

Das Dialkylacetal der Formel (IV) kann aber auch ohne jede weitere Aufreinigung gleich dem zweiten Schritt des erfindungsgemäßen Verfahrens, der Acetalspaltung zugeführt werden.
Die Acetalspaltung wird mittels saurer Katalyse oder in Anwesenheit von Übergangsmetallkatalysatoren, beispielsweise von Lanthanidenkatalysatoren durchgeführt.
Als Katalysator für die saure Katalyse eignen sich organische oder anorganische Säuren wie etwa Schwefelsäure, p-Toluolsulfonsäure, Ameisensäure, Essigsäure Oxalsäure, Amidosulfonsäure u.s.w.
Als Lanthaniden kommen diverse Verbindungen von Cer, Lanthan, Ytterbium, Samarium u.s.w. in Frage. Dies sind insbesondere Chloride, Sulfate und Carboxylate.

Bevorzugt wird die Acetalspaltung unter saurer Katalyse durchgeführt. Besonders bevorzugt wird dazu Schwefelsäure verwendet.

Durch die Zugabe von Wasser und des entsprechenden Katalysators, bevorzugt von katalytischen Mengen an Säure und durch Abdestillieren des abgespaltenen Alkohols wird das Dialkylacetal gespalten und in die gewünschte Verbindung der Formel (I) überführt.
Wasser wird dabei in mindestens äquimolarer Menge bzw. im molaren Überschuss bezogen auf das Acetal eingesetzt.
Die Isolierung des Endproduktes erfolgt durch übliche Methoden, wie etwa Extraktion und nachfolgender destillativer Reinigung.

Durch das erfindungsgemäße Verfahren werden die gewünschten Alkyl- oder Aryloxyacetaldehyde der Formel (I) in einfacher Weise ausgehend von leicht zugänglichen Edukten in hohen Ausbeuten und hoher Reinheit erhalten.

### Beispiel 1:

### a) Herstellung von Benzyloxyacetaldehyddimethylacetal

Es wurden 648 g (6 mol) Benzylalkohol vorgelegt und 3 mol Natriummethylat (30 % in Methanol) zudosiert. Anschließend wurden 474 g Methanol abdestilliert, bei ca. 135°C 374 g (3 mol) Chloracetaldehyddimethylacetal innerhalb 2 Stunden zudosiert und bei dieser Temperatur 2 Stunden nachreagiert. NaCI fiel aus. Nach dem Abkühlen auf 70°C wurden 700 ml Wasser zugegeben und die Phasen getrennt. Anschließend wurde die organische Phase (1011 g) bei 13 mbar über eine Kolonne destillativ aufgearbeitet. 550 g Vorlauf enthielten überschüssigen Benzylalkohol und weitere Leichtsieder, bei 125 bis 128 °C destillierten 396 g Benzyloxyacetaldehyddimethylacetal über (67 % Ausbeute bezogen auf eingesetztes Chloracetaldehyddimethylacetal).

### b) Acetalspaltung zum Benzyloxyacetaldehyd:

Nach dem Versetzen des Acetals (195 g, 1 mol) mit der dreifachen Menge an Wasser, das mit Schwefelsäure auf pH 1 gestellt wurde, wurde bei 70°C und 300 mbar solange Methanol und Wasser abdestilliert bis in der organischen Phase laut GC-Analyse kein Acetal mehr vorhanden war. Anschließend wurde der Benzyloxyacetaldehyd zweimal mit 300 ml Methyl-t.butyl-ether (MtBE) extrahiert. Die organische Phase wurde am Vapsilator von MtBE befreit und der Rückstand anschließend bei 1 mbar und 75°C destilliert. Ausbeute an Benzyloxyacetaldehyd betrug 135 g (90 % auf eingesetztes Acetal).

## Patentansprüche

1. Verfahren zur Herstellung von Alkyl- oder Aryloxyacetaldehyden der Formel 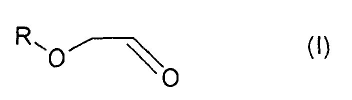 in der R einen gegebenenfalls ein- oder mehrfach substituierten Alkyl-, Aryl-, Heteroaryl-, Alkaryl-, Alkylheteroaryl- oder Aralkylrest oder einen gegebenenfalls ein- oder mehrfach substituierten Heterocyclus oder Alkylheterocyclus bedeuten kann, **dadurch gekennzeichnet, dass** eine Verbindung der Formel
R―OM (II)
in der R wie oben definiert ist und M ein Alkali- oder ein Erdalkaliatom sein kann mit einer Verbindung der Formel 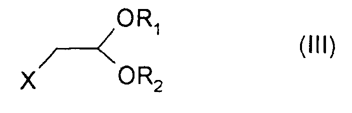 in der R₁ und R₂ unabhängig voneinander einen C₁-C₆-Alkylrest oder gemeinsam einen C₂-C₆-Alkylenrest und X ein Halogenatom bedeuten, zu dem entsprechenden Dialkylacetal der Formel 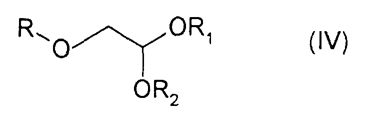 in der R, R₁ und R₂ wie oben definiert sind, umgesetzt wird, worauf eine Acetalspaltung zum gewünschten Alkyl- oder Aryloxyacetaldehyde der Formel (I) durchgeführt wird.

2. Verfahren nach Anspruch 1, **dadurch gekennzeichnet, dass** in der Verbindung der Formel (I) R einen gesättigten oder ein- oder mehrfach ungesättigten, linearen, verzweigten oder cyclischen C₁-C₂₀-Alkylrest, einen C₁-C₂₀-Arylrest oder einen Alkarylrest bedeutet, wobei die Reste gegebenenfalls ein- oder mehrfach durch Carbonsäureester oder Carbonsäureamide, C₁-C₆-Alkoxy, C₆-C₂₀-Aryloxy, Nitro oder Cyano substituiert sein können.

3. Verfahren nach Anspruch 1, **dadurch gekennzeichnet, dass** in der Verbindung der Formel (I) R einen gesättigten, linearen oder verzweigten C₂-C₈-Alkylrest oder einen Benzylrest bedeutet, wobei die Reste gegebenenfalls ein- oder mehrfach durch Carbonsäureester, C₁-C₆-Alkoxy, C₆-C₂₀-Aryloxy, Nitro oder Cyano substituiert sein können.

4. Verfahren nach Anspruch 1, **dadurch gekennzeichnet, dass** in der Formel (II) M Li, Na, K, Ca, Mg oder Cs bedeutet.

5. Verfahren nach Anspruch 1, **dadurch gekennzeichnet, dass** in der Formel (III) R₁ und R₂ unabhängig voneinander einen linearen oder verzweigten C₁-C₄-Alkylrest oder gemeinsam einen C₂-C₄-Alkylenrest bedeuten und X Cl oder Br ist.

6. Verfahren nach Anspruch 1, **dadurch gekennzeichnet, dass** die Verbindungen der Formel (II) und (III) in äquimolarer Menge oder die Verbindung der Formel (II) im molaren Überschuss von 1,1 bis 2 mol pro mol Verbindung der Formel (III) eingesetzt wird.

7. Verfahren nach Anspruch 1, **dadurch gekennzeichnet, dass** die Verbindung der Formel (II) als Lösungsmittel dient.

8. Verfahren nach Anspruch 1, **dadurch gekennzeichnet, dass** die Verbindung der Formel (IV), nach Zugabe von Wasser zum Lösen von eventuell ausgefallenem Salz MX, wobei M und X wie in den Formeln (II) und (III) definiert sind, aus dem Reaktionsgemisch isoliert und der Acetalspaltung zugeführt wird.

9. Verfahren nach Anspruch 1, **dadurch gekennzeichnet, dass** die Acetalspaltung mittels saurer Katalyse mit einer organischen oder einer anorganischen Säure durchgeführt wird.

10. Verfahren nach Anspruch 1, **dadurch gekennzeichnet, dass** zur Acetalspaltung Wasser in mindestens äquimolarer Menge bzw. im molaren Überschuss bezogen auf das Acetal eingesetzt wird.
